(19) Europäisches Patentamt
European Patent Office
Office européen des brevets

(11) **EP 1 052 979 B1**

(12) **EUROPÄISCHE PATENTSCHRIFT**

(45) Veröffentlichungstag und Bekanntmachung des Hinweises auf die Patenterteilung:
**28.09.2005 Patentblatt 2005/39**

(21) Anmeldenummer: **99934198.5**

(22) Anmeldetag: **29.01.1999**

(51) Int Cl.$^7$: **A61K 9/70**

(86) Internationale Anmeldenummer:
**PCT/EP1999/000582**

(87) Internationale Veröffentlichungsnummer:
**WO 1999/039701 (12.08.1999 Gazette 1999/32)**

(54) **VORRICHTUNG ZUR FREIGABE VON STOFFEN**

DEVICE FOR RELEASING SUBSTANCES

SYSTEME POUR LIBERER DES SUBSTANCES

(84) Benannte Vertragsstaaten:
**AT DE ES FR GB IT NL SE**

(30) Priorität: **07.02.1998 DE 19804604**

(43) Veröffentlichungstag der Anmeldung:
**22.11.2000 Patentblatt 2000/47**

(73) Patentinhaber: **Beiersdorf Aktiengesellschaft 20245 Hamburg (DE)**

(72) Erfinder: **RADLOFF, Detlev 20257 - Hamburg (DE)**

(56) Entgegenhaltungen:
**EP-A- 0 914 820       DE-C- 4 224 325
US-A- 5 286 781**

- **CHEMICAL ABSTRACTS, vol. 124, no. 16, 15. April 1996 Columbus, Ohio, US; abstract no. 212122, Seite 709; Spalte 1; XP002106640 & JP 00 803032 A (BANDO CHEMICAL IND.) 9. Januar 1996**
- **DATABASE WPI Week 9050 Derwent Publications Ltd., London, GB; AN 90-372979 XP002106641 & JP 02 270818 A (SEKISUI CHEM IND CO LTD) , 5. November 1990**

**Beschreibung**

Die Erfindung betrifft eine Vorrichtung zur Freigabe von zumindest einem Stoff. Eine besondere Ausführungsform beinhaltet ein Trägermaterial und einer darauf zumindest partiell aufgebrachten Vorrichtung, die als Klebemasse ausgebildet ist, welche den Wirkstoff oder gegebenenfalls auch mehrere Wirkstoffe enthält, die an die Haut abgegeben werden.

Transdermale Therapeutische Systeme (TTS) sind Darreichungsformen von Medikamenten, die einen oder mehrere Arzneistoffe über einen definierten Zeitraum an ihrem Anwendungsort an die Haut abgeben. Es wird dabei zwischen systemisch und lokal wirksamen Darreichungsformen unterschieden.

Bei systemisch wirkenden Darreichungsformen gelangt der Wirkstoff durch Diffusion durch die Haut in den Blutkreislauf und kann im ganzen Körper wirken.

Lokal wirkende Darreichungsformen wirken dagegen nur an den applizierten Stellen. Der Wirkstoff verbleibt in der Haut beziehungsweise in den darunter liegenden Schichten.

Stark klebende Pflaster werden üblicherweise vollflächig mit einer Kautschuk-Klebemasse beschichtet. Das Verkleben derartiger Produkte auf der Haut zeigt nach dem Ablösen deutliche Hautinitationen und eine mechanische Beanspruchung der Haut.

Die Verklebung läßt sich ohne Hilfsmittel nur unter Schmerzen lösen.

Fallweise kommt es zu allergischen Reaktionen. Die verwendeten Klebemassen führen darüber hinaus oft zu einem Massetransfer, d.h. einem Umspulen auf die Haut.

Die Verwendung von hautfreundlichen Klebemassen, wie Acrylatklebemassen und Hydrogele, ist aufgrund der geringen Scherstabilität und Anfaßklebrigkeit nicht erwägenswert. Eine Verbesserung durch eine Nachbehandlung, insbesondere Vernetzung, ist möglich, jedoch bleibt das Ergebnis insgesamt unbefriedigend. Die propriorezeptive Wirkung ist gegenüber den Systemen mit einer Kautschuk-Klebemasse geringer.

Andere bekannte Klebesysteme auf Basis von herkömmlichen Blockcopolymeren sind zum einen durch hohen Stabilisatorzusatz nicht hautfreundlich beziehungsweise zeigen durch die hohe Kohäsivität bislang nur eine Eignung für technische Anwendungsfälle, zum anderen sind sie nicht stark hautklebend und hauthaftend einzustellen.

Die zuvor genannten Klebemassen sind druckempfindliche Selbstklebemassen, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Eine gängige Verarbeitungsweise ist die Schmelze.

Auch solche Haftschmelzklebemassen sind im Stande der Technik bereits vorbeschrieben. Sie basieren auf natürlichen oder synthetischen Kautschuken und/oder anderen synthetischen Polymeren.

Vorteilhaft an den 100 %-Systemen ist, daß bei ihnen verfahrenstechnisch ein Entfernen der Trägermatrix, d.h. der Hilfsmittel, vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Aufgrund ihrer hohen Härte ist für solche 100 %-Systeme die Hauthaftung problematisch.

Es ist ferner bekannt, derartige Selbstklebemassen nicht nur vollflächig sondern auch rasterpunktförmig aufzubringen, beispielsweise durch Siebdruck (DE-PS 42 37 252). wobei die Klebstoffpünktchen auch unterschiedlich groß und/oder unterschiedlich verteilt sein können (EP-PS 353 972), oder durch Tiefdruck von in Längs- und Querrichtung zusammenhängenden Stegen (DE-PS 43 08 649).

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, daß sich bei zu hoher Flächendeckung der an sich undurchlässigen Klebeschicht die Luft- und Wasserdampfdurchlässigkeit entsprechend verringert sowie der Klebemassenverbrauch steigt und bei geringer Flächendeckung der Klebeschicht die Klebeeigenschaften leiden, d.h., das Produkt löst sich, insbesondere bei schweren, textilen Trägermaterialien, zu leicht vom Untergrund.

Im Stande der Technik sind bereits zahlreiche Ausführungsformen wirkstoffhaltiger Pflaster beschrieben worden, die zum Teil nach dem Reservoirprinzip arbeiten, bei welchem der Wirkstoff beispielsweise über eine Membran abgegeben wird, teilweise auch mit einem Matrixsystem oder mit einem komplizierteren mehrschichtigen Aufbau.

Es ist ferner bekannt, daß die Klebemasse des Pflasters als den Wirkstoff enthaltende Matrix eingesetzt werden kann. Neben aus Lösung aufgetragenen Selbstklebemassen wurden auch schon Heißschmelzklebemassen dafür vorgeschlagen, zum Beispiel in der EP-A 663 431, EP-A 452 034, EP-A 305 757, DE-OS 43 10 012, DE-OS 42 22 334 und DE-C 42 24 325. Als Wirkstoffe wurden hierbei, wenn diese benannt wurden, systemisch wirkende aufgeführt.

Beispiele für wirkstoffhaltige Pflaster sind durchblutungsfördernde, antimykotische und keratolytische Wirkstoffpflaster.

Derartige Pflaster, welche fallweise großflächig aufgebracht werden müssen, zeigen jedoch nach dem Ablösen bei empfindlichen Patienten zum Teil deutliche mechanische Hautirritationen. Fallweise kommt es zu allergischen Reaktionen. Ihr Entfernen ist nach längerer Tragezeit bis zu einem gewissen Grade schmerzhaft.

Ein weiterer Nachteil der bekannten wärmewirksamen Pflaster mit einer Klebemasse auf Basis von natürlichem Kautschuk, die in Form einer Lösung mit organischen Lösungsmitteln auf den Pflasterträger aufgetragen wird, ist die vergleichsweise niedrige Freisetzungsrate des Wirkstoffs.

Die geschilderten und weitere Nachteile treffen ebenso auch auf wirkstoffhaltige Pflaster zu, die andere als die genannten Stoffe beinhalten.

So beschreibt die WO 94/02123 ein Wirkstoffpflaster auf Basis von Haftschmelzklebemassen, welche niedrigschmelzende und/oder leicht flüchtige Wirkstoffe in einer Konzentration von 2,5 Gew.-% bis 25 Gew.-% enthalten. Bei den dort eingesetzten Polymeren handelt es sich um A-B-A-Dreiblock Styrol-Ethylen-Butylen-Styrol-Blockcopolymerisate, welche sich durch einen geringen Initialtack und geringe Klebkraft auf Haut auszeichnen.

EP 0 663 431 A2, EP 0 443 759 A3, EP 0 452 034 A2 und US 5,371,128 beschreiben Verwendungen von druckempfindlichen Schmelzhaftklebern auf Silikonbasis mit diversen Additiven und in differenzierten Aufbauformen.

DE 43 10 012 A1 beschreibt den Aufbau eines dermalen therapeutischen Systems aus schmelzfähigen Poly (meth)acrylat-Mischungen.

Insbesondere scheint die Freigabe von mehreren Wirkstoffen aus Polymersystemen, welche nur von einer Polymertype gebildet werden, schwierig. Systeme mit mehreren Polymertypen sind jedoch kritisch in ihrer Verträglichkeit.

DE 43 16 751 C1 beschreibt ein Mehrkammersystem zur Darreichung von Wirkstoffen.

EP 0 439 180 beschreibt ein Wirkstoffpflaster zur Darreichung von Tulobuterol.

EP 0 305 757 beschreibt ein Wirkstoffpflaster zur Darreichung von Nicotin.

EP 0 305 758 beschreibt ein Wirkstoffpflaster zur Darreichung von Nitroglycerin.

EP 0 305 756 beschreibt eine Vorrichtung zur Freisetzung von Stoffen sowie ihre Herstellung und Verwendung.

DE 37 43 945 beschreibt eine Vorrichtung zur Abgabe von Stoffen sowie das Herstellverfahren. Bei der beschriebenen Haftschmelzklebemasse auf Basis von SIS handelt es sich um keine selbstklebende Vorrichtung. Die dort angegebenen Verarbeitungsbereiche liegen deutlich unter denen von Heißschmelzklebemassen und würden für solche beschriebenen Systeme keine ausreichende Verankerung der Klebemasse bieten.

WO 96/22083 zeigt einen Polyisobutylenkleber für transdermale Zwecke, welcher einen Klebrigmacher mit hohem Glasübergangspunkt hat. Der Kleber ist nicht geschäumt.

JP 07-196505 beschreibt eine Darreichung von Inkomethacin in Schmelzhaftklebern. Es wird hier ein Polyethylenschaum als Trägermaterial verwendet.

EP 0 428 017 beschreibt eine Klebstoffkomposition, auf Basis von SEBS und SEPS Blockcopolymeren. Eine Freigabe von lokal oder systemisch wirkenden Stoffen ist jedoch nicht beschrieben.

US 5,085,655 beschreibt die Verwendung von SEPS-Blockcopolymeren als kohäsives Haftsystem insbesondere von Windeln und Slipeinlagen. Die Möglichkeit der Freigabe von Stoffen ist nicht beschrieben.

JP 09 188 865 beschreibt ein System auf Basis von synthetischen Blockpolymeren. Die Freisetzung von Stoffen ist nicht beschrieben.

JP 08277382 beschreibt ein System auf Basis von SEPS, welches als Hotmelt-Klebemasse für Windel und Damenbinden dient. Die Freisetzung von Stoffen ist nicht beschrieben.

JP 08209094 beschreibt ein System auf Basis von synthetischen Blockpolymeren. Die Freisetzung von Stoffen ist nicht beschrieben.

JP 03160083 beschreibt ein System auf Basis von SEPS, welches als Hotmelt-Klebemasse für Windel und Damenbinden dient. Die Freisetzung von Stoffen ist nicht beschrieben.

Aufgabe der Erfindung ist es deshalb, eine wirkstoffhaltige Vorrichtung zur Verfügung zu stellen, welche sich bei Vermeidung der aus dem Stand der Technik bekannten Nachteile durch eine gute Wirksamkeit, d.h. eine relativ hohe Freisetzungsrate, auch bei verschiedenen Wirkstoffkombinationen, und gute Hautverträglichkeit bei gleichzeitig gutem Klebeverhalten auszeichnet. Auch soll die Vorrichtung sich technisch wenig aufwendig und umweltverträglich herstellen lassen.

Gelöst wird diese Aufgabe durch eine Vorrichtung zur Freigabe von Stoffen, wie sie im Hauptanspruch niedergelegt ist. Gegenstand der Unteransprüche sind vorteilhafte Ausführungsformen der erfindungsgemäßen Vorrichtungen. Des weiteren umfaßt die Erfindung auch Verfahren zur Herstellung derartiger Vorrichtungen.

Demgemäß betrifft die Erfindung eine Vorrichtung zur Freigabe von Wirkstoffen, die bei einer Frequenz von 0,1 rad/s eine Glasübergangstemperatur von kleiner 15 °C, einen SEPS-Blockcopolymeranteil von wenigstens 3 Gew.-% aufweist und mindestens einen lokal oder systemisch wirkenden Stoff enthält, in der bevorzugten Ausführungsform unterschiedliche wirkende Stoffe. Der oder die Wirkstoffe sind nicht hyperämisierend, des weiteren sollte die Vorrichtung nicht geschäumt sein.

SEPS steht dabei für Styrol-Ethylen-Propylen-Styrol, bestehend aus einem Blockcopolymeren auf der Basis von Polystyrolblöcken (S) und Blöcken aus hydriertem Polyisopren (EP) oder hydriertem Poly(butadien-co-isopren) (EEP).

Es können Di-, Tri- und Multiblöcke eingesetzt werden.

In einer vorteilhaften Ausführungsform weist die Vorrichtung einen A/B-Diblockanteil von mehr als 30 %, bevorzugt größer 60 %, auf.

Vorzugsweise liegen die Mengenkonzentrationen des oder der Wirkstoffe in der Klebmasse zwischen 0,01 bis ca. 60 Gew.-%, vorzugsweise 0,1 bis 20 Gew.-%.

Unter der Bezeichnung "Wirkstoffe" im Zusammenhang mit der vorliegenden Erfindung werden chemische Elemente, organische und anorganische Verbindungen verstanden, die aus den sie enthaltenden Bestandteilen einer gattungsgemäßen Vorrichtung herauswandern können und dadurch einen angestrebten Effekt hervorrufen. Unter den Anwendungsgebieten der erfindungsgemäßen Vorrichtung ist die Human- und Tiermedizin von besonderer Bedeutung, wobei hier eine Ausgestaltung der Erfindung in Pflasterform besonders bevorzugt ist.

Typische Wirkstoffe sind - ohne den Anspruch der Vollständigkeit zu erheben - für die Herstellung von wirkstoffdotierten Pflastersystemen im Rahmen der vorliegenden Erfindung:

| Indikation: | Wirkstoff |
|---|---|
| Antimykotika | Nafitin |
| | Amorrolfin |
| | Tolnaftat |
| | Ciclopirox |
| Antiseptika | Thymol |
| | Eugenol |
| | Triclosan |
| | Hexachlorophen |
| | Benzalkoniumchlorid |
| | Clioquinol |
| | Chinolinol |
| | Undecensäure |
| | Ethacridin |
| | Chlorhexidin |
| | Hexetidin |
| | Dodicin |
| | Iod |
| Nichtsteroidale Antirheumatika | Glykolsalicylat |
| | Flufenaminsäure |
| | Etofenamat |
| | Ketoprofen |
| | Piroxicam |
| | Indomethacin |
| Antipuriginosa | Polidocanol |
| | Isoprenalin |
| | Crotamiton |
| Lokalanästhetika | Benzocain |
| Antipsoriatika | Ammoniumbitumasulfonat |
| Keratolytika | Harnstoff |

Die Verteilung der Wirkstoffe in der Vorrichtung erfolgt vorzugsweise in einem Thermohomogenisator, wie zum Beispiel Thermomixer, Thermokneter, Walzenwerke oder Schneckensystemen. Die Zugabe des Wirkstoffs kann in die vollständig hergestellte Vorrichtung erfolgen. Beispielhaft kann der Wirkstoff auch in eine Zwischenstufe oder in die Ausgangsmischung eingearbeitet werden.

Vorteilsweise handelt es sich bei der Vorrichtung um eine Klebemasse, welche in einer besonders bevorzugten Ausführung eine Heißschmelzklebemasse mit einem SEPS-Blockcopolymeranteil von wenigstens 3 Gew.-% ist. In einer bevorzugten Ausführung werden Dreiblockcopolymere eingesetzt. Für spezielle Vorrichtungen sind SEP-Diblock oder Multiblockpolymere vorteilhaft.

Je nach Einsatzgebiet lassen sich die klebetechnischen Eigenschaften einstellen. Fallweise werden stark selbstklebende System oder aber auch haftklebrige Systeme benötigt. Hierzu werden dem Polymersystem entsprechende Zusatzstoffen wie Klebharze, Weichmacher, Stabilisatoren und andere Hilfsstoffen zugesetzt.

Ihr Erweichungspunkt sollte höher als 50 °C liegen, da die Applikationstemperatur in der Regel mindestens 70 °C beträgt, bevorzugt zwischen 90 °C und 150 °C. Gegebenenfalls kann eine Nachvemetzung durch UV- oder Elektronenstrahlen-Bestrahlung angebracht sein. Dieses hängt von dem speziellen Aufbau des Stammpolymers oder deren Zusatzstoffen ab.

Besonders vorteilhaft ist, wenn weitere Polymere in der Vorrichtung enthalten sind, welche bevorzugt auf Blockcopolymeren basieren. Insbesondere die Abmischung von Blockcopolymeren auf Basis von SEPS und SEBS zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzselbstklebemasse wird durch den kohäsiven Charakter des Polymeren erreicht. Die gute Anfaßklebrigkeit ergibt sich durch die eingesetzte Palette an Klebrigmachern und Weichmachern.

Für besonders starkklebende Systeme basiert die Heißschmelzselbstklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält wenigstens eine weiche Phase B auf Basis von Ethylen, Butylen, Propylen, Isopren, Butadien oder deren Mischungen.

Weiter vorzugsweise sind die Polymere sowie die Vorrichtung auf Basis von Di-(A-B)- und/oder Tri-(ABA)-Blockcopolymeren aufgebaut mit einem Anteil an Di-Blockcopolymeren von weniger als 80 Gew.-%.

Die Kette der Phase B kann auch artfremde Teile enthalten, wie zum Beispiel Isopren, Butadien oder ähnliche Stoffe. Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 3 Gew.-% und 30 Gew.-%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

Weiter kann die Klebemasse auch Hilfsstoffe zur besonderen Freigabe oder deren Unterstützung beinhalten. Beispielhaft sind hier Polypropylenglycol oder Polyethylenglycol genannt.

In einer vorteilhaften Ausführung weist die Heißschmelzselbstklebemasse mit einem SEPS-Blockcopolymeranteil von wenigstens 3 Gew.-% die nachfolgend angegebene Zusammensetzung auf:

| | |
|---|---|
| 3 Gew.-% bis 90 Gew.-% | Blockcopolymere, |
| 5 Gew.-% bis 80 Gew.-% | Klebrigmacher wie aliphatische Kohlenwasserstoffharze, hydrierte Terpenharze, hydrierte Kohlenwasserstoffharze, Cyclopentadienharze, Styrol-$\alpha$-methylstyrol Harze und niedermolekulare Polyisobutylene, |
| weniger als 60 Gew.-% | Weichmacher wie Paraffinöle, aliphatische KW-Öle, Wachse und Fettsäureester und -alkoholether, |
| weniger als 15 Gew.-% | Additive, |
| weniger als 20 Gew.-% | Hilfsstoffe zur Freigabe der Wirkstoffe, |
| weniger als 5 Gew.-% | Stabilisatoren, |
| weniger als 60 Gew.-% | Wirkstoff oder Wirkstoffe. |

Die Klebrigmacher und Weichmacher dienen dabei der Einstellung der Klebeeigenschaften und der Stabilität. Gegebenenfalls kommen weitere Stabilisatoren und andere Hilfsstoffe zum Einsatz.

Beispielhaft sind folgende Polymer/Klebrigmacherkombinationen genannt.

| | Typ | E1 | E2 | E3 |
|---|---|---|---|---|
| SEPS (Kuraray Co.) | T1 | 30 | 10 | |
| Styrol-Anteil 13 % | T2 | | 20 | 40 |
| SEBS (Shell) | T3 | 5 | | |
| Styrol-Anteil 30 % | T4 | | 5 | |
| Kohlenwasserstoffharz | | 40 | 49 | 40 |
| Kohlenwasserstofföl | | 24 | 15 | 19 |
| Antioxidanz | | 1 | 1 | 1 |

Kombinationen anderer Polymersysteme sind für den Fachmann naheliegend.

Ein Füllen der Klebemasse mit mineralischen Füllstoffen, Fasern, Mikrohohl- oder -vollkugeln ist möglich.

Die Heißschmelzselbstklebemasse weist einen Erweichungspunkt auf oberhalb von 50 °C, bevorzugt 70 °C bis 220 °C, ganz besonders bevorzugt 75 °C bis 140 °C.

Die dynamische Glasübergangstemperatur der Elastomerphase der haftklebrigen Matrix beträgt bei einer Frequenz von 0,1 rad/s weniger als 15 °C, bevorzugt von -3 °C bis - 30 °C, ganz besonders bevorzugt von -9 °C bis -25 °C.

Insbesondere an Pflaster werden hohe Anforderungen bezüglich der Klebeeigenschaften gestellt. Für eine ideale Anwendung sollte die Heißschmelzselbstklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzselbstklebemasse notwendig.

Durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut und der Trägerrückseite erreicht.

Die hohe Scherfestigkeit der hier eingesetzten Selbstklebemasse wird durch die hohe Kohäsivität des Blockcopolymeren erreicht. Die gute Anfaßklebrigkeit ergibt sich durch die eingesetzte Palette an Klebrigmachern und Weichmachern.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils.

Hierbei wird bei einer vorgegebenen Temperatur die Heißschmelzselbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient (Q = tan δ) zwischen dem Verlustmodul (G" viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Der komplexe-dynamische Glasübergangsbereich ist der Übergangsbereich vom amorphen in den viskoelastischen Bereich. Er entspricht dem Maximum der Temperaturfunktion bei vorgegebener Frequenz.

| Bezeichnung | $T_G$ niedrige Frequenz | Anschmiegsamkeit niedrige Frequenz/RT | Anfaßklebrigkeit hohe Frequenz/RT |
|---|---|---|---|
| Selbstklebemasse A | 4 ± 2 °C | tan δ = 0,08 ± 0,03 | tan δ = 0,84 ± 0,03 |
| Selbstklebemasse B | -9 ± 2 °C | tan δ = 0,32 ± 0,03 | tan δ = 1,70 ± 0,03 |

Bevorzugt werden erfindungsgemäß Heißschmelzselbstklebemassen, bei denen das Verhältnis des viskosen Anteils zum elastischen Anteil bei einer Frequenz von 100 rad/s bei 25 °C größer 0,7 ist, oder Heißschmelzselbstklebemassen, bei denen das Verhältnis des viskosen Anteils zum elastischen Anteil bei einer Frequenz von 0,1 rad/s bei 25 °C kleiner 0,6 ist, bevorzugt zwischen 0,35 und 0,02, ganz besonders bevorzugt zwischen 0,3 und 0,1.

In Ausnahmefällen kann eine abgewandelte Verwendung der Vorrichtung durch das Schäumen dieser erreicht werden.

Die mit den Wirkstoffen versehenen Vorrichtungen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100 °C und vergleichsweise hohem Innendruck gearbeitet, entstehen sehr offenporige Klebstoffschaumschichten, die besonders gut luft- und wasserdampfdurchlässig sind. Die vorteilhaften Eigenschaften der Vorrichtung sind die gute Anschmiegsamkeit auch an unebenen Flächen durch die Elastizität und Plastizität der geschäumten Vorrichtung

Gleichzeitig wird durch die Vakuolen im Schaum der Transport der Wirkstoffe überproportional gesteigert, womit sehr gute Freisetzungsraten erreicht werden.

Ein besonders geeignetes Verfahren zur Herstellung der erfindungsgemäß geschäumten Vorrichtung arbeitet nach dem Schaum-Mix-System. Hierbei wird die thermoplastische Klebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt. Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Extruder- oder Kammersysteme Verwendung.

Die Wechselwirkung der Wirkstoffe mit der Haut wird bekanntlich mit in die Klebemasse eingemischten Enhancern moduliert beziehungsweise durch den okklusiven Effekt von Klebemasse und Abdeckung verstärkt. Im Gegensatz hierzu kann durch den Einsatz von atmungsaktiven dotierten Beschichtungen in Verbindung mit elastischen ebenfalls atmungsaktiven Trägermaterialien ein insbesondere zum Beispiel während sportlicher Betätigungen

a) vom Anwender subjektiv angenehmer empfundener Tragekomfort und

b) vom Freisetzungsverhalten durch die mit der Umgebung ungestörtere Wechselwirkung (zum Beispiel Unterdrückung von Körperschweiß) der Haut eine definierter Penetration von Wirkstoffen in die Haut erzielt werden.

Umgekehrt läßt sich durch die hier genannten Verfahren auch eine Durchlässigkeit des dotierten Pflastersystems von außen erzielen. Mit dieser Produkteigenschaft können somit von außen durch den Träger Substanzen an die Kontaktstelle dotierter Kleber/Haut auch zu späteren Zeiten nach der eigentlichen Applikation herangebracht werden (Flüssigkeit aufträufeln, wischen, etc.). Diese Substanzen könnten zum Beispiel eine zusätzliche Enhancerwirkung beinhalten oder die medikamentöse Wirkung starten, schwächen oder für eine entsprechende Konsumentenauslobung geeignet modulieren.

Durch die Schäumung der Vorrichtung und die dadurch entstandenen offenen Poren in der Masse sind bei Verwendung eines an sich porösen Trägers die mit der Vorrichtung beschichteten Produkte gut wasserdampf- und luftdurchlässig. Die benötigte Vorrichtungsmenge wird erheblich reduziert ohne Beeinträchtigung und Wirkungsweise der Vorrichtungseigenschaften.

Durch das Schäumen wird zudem die Viskosität der Vorrichtung in der Regel gesenkt. Hierdurch wird die Schmelzenergie erniedrigt und es können auch thermoinstabile Trägermaterialien direkt beschichtet werden.

Vorteilhaft insbesondere für die Verwendung bei medizinischen Produkten ist weiterhin, wenn die dotierte Masse partiell auf dem Trägermaterial aufgetragen ist, beispielsweise durch Rasterdruck, Thermosiebdruck, Thermoflexodruck oder Tiefdruck, denn im Vollstrich selbstklebend ausgerüstete Trägermaterialien können unter ungünstigen Voraussetzungen bei der Applikation mechanische Hautirritationen hervorrufen.

Ferner kann die Klebemasse beispielsweise auch aufgesprüht werden, was ein mehr oder weniger unregelmäßiges Auftragsbild ergibt.

Der partielle Auftrag ermöglicht durch geregelte Kanäle die Abführung des transepidermalen Wasserverlustes und verbessert das Ausdampfen der Haut beim Schwitzen insbesondere bei der Verwendung von luft- und wasserdampfdurchlässigen Trägermaterialien. Hierdurch werden Hautirritationen, die durch Stauungen der Körperflüssigkeiten hervorgerufen werden, vermieden. Die angelegten Abführungskanäle ermöglichen ein Ableiten.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.

Die dotierte Masse kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der bevorzugten Masse beschickt wird. Eine speziell geformte Düsenlippe (Rund- oder Vierkantrakel) preßt die über einen Kanal eingespeiste Masse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen dotierten Kalotten geschieht dabei nach folgendem Mechanismus:

Der Düsenrakeldruck fördert die dotierte Masse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Selbstklebemasse und die innere Kohäsion des Hotmelts wird von der auf dem Träger bereits haftenden Basis der Kalotten der in den Löchern begrenzte Vorrat an Heißschmelzselbstklebemasse konturenscharf abgezogen beziehungsweise durch den Rakeldruck auf den Träger gefördert.

Nach Beendigung dieses Transportes formt sich, abhängig von der Rheologie der Heißschmelzselbstklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 3:1 sein, bevorzugt kleiner oder gleich 1:1, insbesondere gleich 1:3.

Der beschriebene Bildungsmechanismus der Kalotten erfordert bevorzugt saugfähige oder zumindest von der dotierten Masse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.

Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 220 m/min, beschichtet, wobei die Beschichtungstemperatur größer als die Erweichungstemperatur zu wählen ist.

Die dotierte Vorrichtung kann mit einem Flächengewicht von größer 15 g/m², bevorzugt zwischen 90 g/m² und 400 g/m², ganz besonders bevorzugt zwischen 130 g/m² und 300 g/m², auf dem Trägermaterial aufgetragen sein.

Der prozentuale Anteil, der mit der dotierten Vorrichtung beschichteten Fläche sollte mindestens 20 % betragen und kann bis zu ungefähr 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Vorrichtungen mit unterschiedlichen Eigenschaften und Dotierungen eingesetzt werden können.

Die Kombination der dotierten Vorrichtungen und der partiellen Beschichtung sichert auf der einen Seite eine sichere Verklebung des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität der Vorrichtung vernachlässigbar, weil die Vorrichtung nicht an Haut und Haar verankert, vielmehr ist die Verankerung der Vorrichtung auf dem Trägermaterial mit bis zu 12 N/cm (Probenbreite) für medizinische Anwendungen gut.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Steuerung, welche eine nachweisliche Absenkung der Haftkraft dieser Vorrichtung erwirkt, die Ablösbarkeit.

Je nach Trägermaterial und dessen Temperaturempfindlichkeit kann Masse direkt aufgetragen sein oder zuerst auf einen Hilfsträger aufgebracht und dann auf den endgültigen Träger transferiert werden.

Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Weiterhin kann eine Behandlung der Masse mit einer Elektronenstrahl-Nachvernetzung oder einer UV-Bestrahlung zu einer Verbesserung der gewünschten Eigenschaften führen.

Als Trägermaterialien eignen sich alle starren und elastischen Flächengebilde aus synthetischen und natürlichen Rohstoffen. Bevorzugt sind Trägermaterialien die nach Applikation der Klebemasse so eingesetzt werden können, daß sie Eigenschaften eines funktionsgerechten Verbandes erfüllen.

Beispielhaft sind Textilien wie Gewebe, Gewirke, Gelege, Vliese, Laminate, Netze, Folien, Schäume und Papiere aufgeführt. Weiter können diese Materialien vorbeziehungsweise nachbehandelt werden. Gängige Vorbehandlungen sind Corona und Hydrophobieren; geläufige Nachbehandlungen sind Kalandern, Tempern, Kaschieren, Stanzen und Eindecken.

Das mit der Masse beschichtete Trägermaterial kann eine Luftdurchlässigkeit von größer 1 $cm^3/(cm^2*s)$, bevorzugt größer 15 $cm^3/(cm^2*s)$, ganz besonders bevorzugt größer 70 $cm^3/(cm^2*s)$, aufweisen, des weiteren eine Wasserdampfdurchlässigkeit von größer 500 $g/(m^2*24h)$, bevorzugt größer 1000 $g/(m^2*24h)$, ganz besonders bevorzugt größer 2000 $g/(m^2*24h)$.

Schließlich kann die Vorrichtung nach dem Beschichtungsvorgang mit einem klebstoffabweisenden Trägermaterial, wie silikonisiertes Papier, eingedeckt oder mit einer Wundauflage oder einer Polsterung versehen werden. Anschließend werden die Vorrichtung in der gewünschten Größe ausgestanzt.

Besonders vorteilhaft ist, wenn die Vorrichtung sterilisiert, bevorzugt γ-(gamma) sterilisiert, ist. Dieses ist besonders geeignet für eine nachträgliche Sterilisation eines Polymersystems auf Blockcopolymerbasis, welche keine Doppelbindungen enthalten. Dieses gilt ins besondere für das erfindungsgemäße Styrol-Propylen-Ethylen-Styrol-Blockcopolymerisate. Es treten hierbei keine für die Anwendung signifikanten Änderungen in den Eigenschaften auf.

Die erfindungsgemäße Vorrichtung kann eine Klebkraft von mindestens 1,5 N/cm aufweisen, besonders eine Klebkraft zwischen 2,5 N/cm und 5 N/cm. Auf anderen Untergründen können höhere Klebkräfte erreicht werden.

Im folgenden sollen besonders vorteilhafte Ausführungsformen der Erfindung beschrieben werden, ohne die Erfindung dadurch unnötig einschränken zu wollen.

Erfindungsgemäß wurden Beispielrezepturen zur Freigabe verschiedener Wirkstoffe hergestellt. Alle Rezepturen wurden lösungsmittelfrei in einem Laborkneter bei einer Temperatur zwischen 90 °C und 180 °C hergestellt. Die Zugabe des pharmazeutischen Wirkstoffes erfolgte während der Abkühlphase bei einer Temperatur, die im Fall von festen Arzneistoffen ca. 10 °C oberhalb des jeweiligen Schmelzpunktes lag. Die Herstellung von Labormustern zur Ausprüfung der Rezepturen wurde mit Hilfe einer Heißpresse durchgeführt.

**Beispiel 1: 5 Gew.-%ige Wirkstoffzubereitung**

39,0 g Septon 4033 (Kuraray), 57,0 g Regalite 91 (Hercules), 46,5 g Hercules MBG 274 (Hercules) und 1,4 g Irganox 1010 (Ciba) wurden in einem Laborkneter bei 150 °C aufgeschmolzen und über einen Zeitraum von ca. 90 min homogenisiert. Anschließend wurden bei 100 °C 7,5 g Ibuprofen zugegeben und weitere 60 min homogenisiert. Die heiße Masse wurde abschließend in eine silikonisierte Wanne gegossen. Zur Herstellung von Labormustern wurden 10,0 g der abgekühlten Masse in einer Schmelzpresse bei 100 °C zwischen zwei silikonisierten Trennpapierblättern auf eine Schichtdicke von 400 μm ausgepreßt. Anschließend wurde nach Entfernen des Trennpapieres eine Seite der Klebeschicht mit silikonisierter Polyesterfolie kaschiert. Auf der gegenüberliegenden Seite wurde eine adhäsiv ausgerüstete Polyesterfolie laminiert. Aus diesem Muster wurden die Proben zur Bestimmung der Klebkraft und der Wirkstofffreisetzung vorbereitet.

Es entstand ein farbloses, transparentes Muster mit hervorragender Klebkraft und sehr guter Kohäsion. Die Klebkraft gemessen auf PMMA (Polymethylmethacrylat) im 90° Peel Test betrug 18,7 N/cm bei einer Abzuggeschwindigkeit von 300 mm/min.

Die Wirkstofffreisetzung wurde mittels einer Keshary-Chien Zelle unter Verwendung einer Silikonmembran und einem wäßrigen Phosphatpuffer als Rezeptorphase bestimmt. Über einen Zeitraum von 24 h wurden 281 $\mu g/cm^2$ des eingearbeiteten Wirkstoffes freigesetzt.

**Beispiele 2 bis 6: 5 Gew.-%ige Wirkstoffzubereitung**

Die folgenden Beispielrezepturen wurden analog zur Vorgehensweise von Beispiel 1 hergestellt.

| Beispiel | Wirkstoff | Zugabe bei |
|---|---|---|
| 2 | Salicylsäure | 160 °C |

(fortgesetzt)

| Beispiel | Wirkstoff | Zugabe bei |
|----------|-----------|------------|
| 3 | Diclofenac | 160 °C |
| 4 | Benzocain | 100 °C |
| 5 | Lidocain*HCl | 130 °C |
| 6 | Bufexamac | 160 °C |

**Beispiele 7 bis 9: 1 Gew.-%ige Wirkstoffzubereitung**

40,7 g Septon 4033 (Kuraray), 59,4 g Regalite 91 (Hercules), 48,4 g Hercules MBG 274 (Hercules) und 1,4 g Irganox 1010 (Ciba) wurden in einem Laborkneter bei 150 °C aufgeschmolzen und über einen Zeitraum von ca. 90 min homogenisiert. Anschließend wurden bei der in der Tabelle angegebenen Temperatur 1,5 g des jeweiligen Wirkstoffes zugegeben und weitere 60 min homogenisiert.
Die weitere Verarbeitung verlief nach der unter Beispiel 1 beschriebenen Vorgehensweise.

| Beispiel | Wirkstoff | Zugabe bei |
|----------|-----------|------------|
| 7 | Clotrimazol | 150 °C |
| 8 | Econazol | 100 °C |
| 9 | Diphenhydramin *HCl | 170 °C |

**Beispiel 10: 1 Gew.-%ige Wirkstoffzubereitung**

Erfindungsgemäß wurde eine ungeschäumte Vorrichtung zur Freigabe von Stoffen hergestellt, welche einen nicht-hyperämisieren Wirkstoff enthielt. Als Wirkstoff wurde Ibuprofen verwendet.
Das Trägermaterial bestand aus einem unelastischen Baumwollgewebe mit einer Höchstzugkraft von größer 80 N/cm und einer Höchstzugkraft-Dehnung von kleiner 20 %.
Diese Schmelzhaftklebemasse setzte sich wie folgt zusammen:

- ein SEPS Blockcopolymer, welches aus harten und weichen Segmenten besteht und einem Styrolgehalt im Polymer von 13 Mol.-%; der Anteil an der Klebemasse beträgt 35 Gew.-% (Kuraray Co.)
- ein Paraffinkohlenwasserstoffharz mit einem Anteil an der Klebmasse von 63 Gew.-%
- ein Alterungsschutzmittel mit einem Anteil von weniger als 1,0 Gew.-% (Irganox 1010 Ciba)
- Ibuprofen mit einem Anteil von 1 Gew.-%

Die eingesetzten Kleberkomponenten wurden in einem Thermomischer bei 165 °C 1,5 Stunden homogenisiert. Der Wirkstoff wurde in der Abkühlphase bei 100°C zugegeben und weitere 75 min im Mischer homogenisiert. Die Selbstklebemasse wurde mit einer Düse vollflächig auf den Träger appliziert. Die direkte Beschichtung erfolgte mit 50 m/min bei einer Temperatur von 120 °C. Das Trägermaterial wurde mit 170 g/m$^2$ beschichtet.
Das so hergestellte Pflastermaterial zeigt eine vergleichbar gute Freisetzung (Liberationsstudie) des Wirkstoffs.
Nach der Applikation wurden keine Hautirrtationen festgestellt.

**Patentansprüche**

1. Vorrichtung zur Freigabe von Wirkstoffen, **dadurch gekennzeichnet, daß** die Vorrichtung bei einer Frequenz von 0,1 rad/s eine Glasübergangstemperatur von kleiner 15 °C, einen SEPS-Blockcopolymeranteil von wenigstens 3 Gew.-% aufweist und mindestens einen lokal oder systemisch wirkenden Stoff enthält, der nicht hyperämisierend wirkt, wobei die Vorrichtung nicht geschäumt ist.

2. Vorrichtung zur Freigabe von Stoffen gemäß Anspruch 1, **dadurch gekennzeichnet, daß** die Vorrichtung unterschiedliche wirkende Stoffe enthält.

**3.** Vorrichtung zur Freigabe von Stoffen gemäß mindestens einem vorangegangenen Anspruch, **dadurch gekennzeichnet, daß** die Vorrichtung den oder die Wirkstoffe in einer Menge von 0,01 bis 60 Gew.-%, bevorzugt von 0,1 bis 20 Gew.-%, enthält.

**4.** Vorrichtung zur Freigabe von Stoffen gemäß mindestens einem vorangegangenen Anspruch, **dadurch gekennzeichnet, daß** die Vorrichtung einen A/B-Diblockanteil von mehr als 30 %, bevorzugt größer 60 % aufweist.

**5.** Vorrichtung zur Freigabe von Stoffen gemäß mindestens einem vorangegangenen Anspruch, **dadurch gekennzeichnet, daß** weitere Polymere in der Vorrichtung enthalten sind, welche bevorzugt auf Blockcopolymeren basieren.

**6.** Vorrichtung zur Freigabe von Stoffen gemäß Anspruch 5, **dadurch gekennzeichnet, daß** die weiteren Polymere wenigstens eine harte Phase A auf Basis von Styrol oder dessen Derivaten und wenigstens eine weiche Phase B auf Basis von Ethylen, Butylen, Propylen, Isopren, Butadien oder deren Mischungen, bevorzugt Ethylen und Butylen, besitzen.

**7.** Vorrichtung zur Freigabe von Stoffen gemäß Anspruch 5, daß die Polymere auf Basis von Di-(A-B)- und/oder Tri-(ABA)-Blockcopolymeren aufgebaut sind mit einem Anteil an Di-Blockcopolymeren von weniger als 80 Gew.-%.

**8.** Vorrichtung zur Freigabe von Stoffen gemäß Anspruch 5, **dadurch gekennzeichnet, daß** der gesamte Styrolanteil im Polymersystem niedriger als 35 Gew.-%, bevorzugt 5 Gew.-% bis 30 Gew.-%, ist.

**9.** Vorrichtung zur Freigabe von Stoffen gemäß mindestens einem der vorangegangenen Anspruch, **dadurch gekennzeichnet, daß** die Vorrichtung klebend, bevorzugt selbstklebend ist.

**10.** Vorrichtung zur Freigabe von Stoffen gemäß mindestens einem vorangegangenen Anspruch, **dadurch gekennzeichnet, daß** die Vorrichtung eine Heißschmelzklebemasse ist mit einem SEPS-Blockcopolymeranteil von wenigstens 3 Gew.-%, bestehend insbesondere aus

| a) | 3 Gew.-% bis 90 Gew.-% | Blockcopolymere, |
|---|---|---|
| b) | 5 Gew.-% bis 80 Gew.-% | Klebrigmacherwiealiphatische Kohlenwasserstoffharze, hydrierte Terpenharze, hydrierte Kohlenwasserstoffharze, Cyclopentadienharze, Styrol$\alpha$-methylstyrol Harze und niedermolekulare Polyisobutylene, |
| c) | weniger als 60 Gew.-% | Weichmacher wie Paraffinöle, aliphatische KW-Öle, Wachse und Fettsäureester und -alkoholether, |
| d) | weniger als 15 Gew.-% | Additive, |
| e) | weniger als 5 Gew.-% | Stabilisatoren, |
| f) | weniger als 20 Gew.-% | Hilfsstoffe zur Freigabe des Wirkstoffs oder der Wirkstoffe, |
| f) | weniger als 60 Gew.-% | Wirkstoff oder Wirkstoffe. |

**11.** Vorrichtung zur Freigabe von Stoffen gemäß mindestens einem vorangegangenen Anspruch, **dadurch gekennzeichnet, daß** die dynamische Glasübergangstemperatur der Elastomerphase der haftklebrigen Matrix bei einer Frequenz von 0,1 rad/s weniger als 15 °C, bevorzugt von -3 °C bis -30 °C, ganz besonders bevorzugt von - 9 °C bis -25 °C, beträgt.

**12.** Vorrichtung zur Freigabe von Stoffen gemäß mindestens einem der vorangegangenen Anspruch, **dadurch gekennzeichnet, daß** die Vorrichtung partiell auf einem Trägermaterial aufgetragen wird, insbesondere durch Rasterdruck, Thermosiebdruck, Thermoflexodruck oder Tiefdruck.

**13.** Vorrichtung zur Freigabe von Stoffen gemäß mindestens einem der vorangegangenen Anspruch, **dadurch gekennzeichnet, daß** die Vorrichtung auf ein Trägermaterial aufgesprüht ist.

**14.** Vorrichtung zur Freigabe von Stoffen gemäß mindestens einem der vorangegangenen Anspruch, **dadurch gekennzeichnet, daß** die Vorrichtung in Form von polygeometrischen Kalotten auf das Trägermaterial gebracht wird.

**15.** Vorrichtung zur Freigabe von Stoffen gemäß mindestens einem der vorangegangenen Anspruch, **dadurch gekennzeichnet, daß** die Vorrichtung mit einem Flächengewicht von größer 15 g/m$^2$, bevorzugt zwischen 90 g/m$^2$

und 400 g/m$^2$, ganz besonders bevorzugt zwischen 130 g/m$^2$ und 300 g/m$^2$, auf dem Trägermaterial aufgetragen ist.

16. Vorrichtung zur Freigabe von Stoffen gemäß mindestens einem der vorangegangenen Anspruch, **dadurch gekennzeichnet, daß** der prozentuale Anteil der mit der Vorrichtung beschichteten Fläche auf dem Trägermaterial mindestens 20 % beträgt, bevorzugt 40 % bis 60 %, ganz besonders bevorzugt 70 % bis 95 %.

17. Vorrichtung zur Freigabe von Stoffen gemäß mindestens einem der vorangegangenen Anspruch, **dadurch gekennzeichnet, daß** das mit der Vorrichtung beschichtete Trägermaterial eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), und eine Wasserdampfdurchlässigkeit von größer 500 g/(m$^2$*24h), bevorzugt größer 1000 g/(m$^2$*24h), ganz besonders bevorzugt größer 2000 g/(m$^2$*24h) aufweist.

18. Vorrichtung zur Freigabe von Stoffen gemäß mindestens einem der vorangegangenen Anspruch, **dadurch gekennzeichnet, daß** die Vorrichtung sterilisiert, bevorzugt γ (gamma)-sterilisiert, ist.

19. Vorrichtung zur Freigabe von Stoffen gemäß mindestens einem der vorangegangenen Anspruch, **dadurch gekennzeichnet, daß** die Vorrichtung eine Klebkraft von mindestens 1,5 N/cm, besonders eine Klebkraft zwischen 2,5 N/cm und 5 N/cm, aufweist.

## Claims

1. Device for releasing active substances, **characterized in that** the device has a glass transition temperature of less than 15°C at a frequency of 0.1 rad/s and has an SEPS block copolymer content of at least 3% by weight, and comprises at least one locally or systemically active substance which does not have a hyperaemic action, the device not being foamed.

2. Substance release device according to Claim 1, **characterized in that** the device comprises different active substances.

3. Substance release device according to either of the preceding claims, **characterized in that** the device comprises the active substance or substances in an amount of from 0.01 to 60% by weight, preferably from 0.1 to 20% by weight.

4. Substance release device according to at least one of the preceding claims, **characterized in that** the device has an A/B diblock fraction of more than 30%, preferably greater than 60%.

5. Substance release device according to at least one of the preceding claims, **characterized in that** the device comprises further polymers which are preferably based on block copolymers.

6. Substance release device according to Claim 5, **characterized in that** the further polymers possess at least one hard phase A based on styrene or derivatives thereof and at least one soft phase B based on ethylene, butylene, propylene, isoprene, butadiene or mixtures thereof, preferably ethylene and butylene.

7. Substance release device according to Claim 5, **characterized in that** the polymers are constructed on the basis of diblock (A-B) and/or triblock (ABA) copolymers, with a proportion of diblock copolymers of less than 80% by weight.

8. Substance release device according to Claim 5, **characterized in that** the overall styrene content in the polymer system is less than 35% by weight, preferably from 5 to 30% by weight.

9. Substance release device according to at least one of the preceding claims, **characterized in that** the device is adhesive, preferably self-adhesive.

10. Substance release device according to at least one of the preceding claims, **characterized in that** the device is a hotmelt adhesive composition having a SEPS block copolymer proportion of at least 3% by weight and consisting in particular of

a) from 3 to 90% by weight of block copolymers,

b) from 5 to 80% by weight of tackifiers, such as aliphatic hydrocarbon resins, hydrogenated terpene resins, hydrogenated hydrocarbon resins, cyclopentadiene resins, styrene-$\alpha$-methylstyrene resins and low molecular mass polyisobutylenes,

c) less than 60% by weight of plasticizers, such as paraffin oils, aliphatic hydrocarbon oils, waxes and fatty acetates and alcoholates,

d) less than 15% by weight of additives,

e) less than 5% by weight of stabilizers,

f) less than 20% by weight of active substance release auxiliaries, and

g) less than 60% by weight of active substance or substances.

**11.** Substance release device according to at least one of the preceding claims, **characterized in that** the dynamic glass transition temperature of the elastomer phase of the adhesive matrix is at a frequency of 0.1 rad/s less than 15°C, preferably from -3 to -30°C and, with very particular preference, from -9 to -25°C.

**12.** Substance release device according to at least one of the preceding claims, **characterized in that** the device is applied partially to a backing material by means, in particular, of halftone printing, thermal screen printing, thermal flexographic printing or intaglio printing.

**13.** Substance release device according to at least one of the preceding claims, **characterized in that** the device is applied to a backing material by spraying.

**14.** Substance release device according to at least one of the preceding claims, **characterized in that** the device is applied in the form of polygeometric domes to the backing material.

**15.** Substance release device according to at least one of the preceding claims, **characterized in that** the device has been applied to the backing material with a weight per unit area of greater than 15 g/m$^2$, preferably between 90 and 400 g/m$^2$ and, with very particular preference, between 130 and 300 g/m$^2$.

**16.** Substance release device according to at least one of the preceding claims, **characterized in that** the percentage area on the backing material that is coated with the device is at least 20%, preferably from 40 to 60% and, with very particular preference, from 70 to 95%.

**17.** Substance release device according to at least one of the preceding claims, **characterized in that** the backing material coated with the device has an air permeability of greater than 1 cm$^3$/(cm$^2$*s), preferably greater than 15 cm$^3$/(cm$^2$*s) and, with very particular preference, greater than 70 cm$^3$/(cm$^2$*s), and a water vapour permeability of greater than 500 g/(m$^2$*24 h), preferably greater than 1000 g/(m$^2$*24 h) and, with very particular preference, greater than 2000 g/(m$^2$*24 h).

**18.** Substance release device according to at least one of the preceding claims, **characterized in that** the device is sterilized, preferably by means of $\gamma$ (gamma) radiation.

**19.** Substance release device according to at least one of the preceding claims, **characterized in that** the device has a bond strength of at least 1.5 N/cm, in particular a bond strength of between 2.5 and 5 N/cm.

**Revendications**

**1.** Dispositif pour la libération de substances actives, **caractérisé en ce que** le dispositif présente, à une fréquence de 0,1 rad/s, une température de transition vitreuse inférieure à 15°C, une proportion de copolymère à blocs de SEPS d'au moins 3% en poids et contient au moins une substance à action locale ou systémique, qui n'a pas d'effet congestionnant, le dispositif n'étant pas moussé.

**2.** Dispositif pour la libération de substances selon la revendication 1, **caractérisé en ce que** le dispositif contient des substances présentant des actions différentes.

**3.** Dispositif pour la libération de substances selon au moins une revendication précédente, **caractérisé en ce que** le dispositif contient la ou les substances actives en une quantité de 0,01 à 60% en poids, de préférence de 0,1 à 20% en poids.

4. Dispositif pour la libération de substances selon au moins une revendication précédente, **caractérisé en ce que** le dispositif présente une proportion de blocs binaires A/B supérieure à 30%, de préférence supérieure à 60%.

5. Dispositif pour la libération de substances selon au moins une revendication précédente, **caractérisé en ce que** d'autres polymères sont contenus dans le dispositif qui sont de préférence à base de copolymères à blocs.

6. Dispositif pour la libération de substances selon la revendication 5, **caractérisé en ce que** les autres polymères présentent au moins une phase dure A à base de styrène ou ses dérivés et au moins une phase souple B à base d'éthylène, de butylène, de propylène, d'isoprène, de butadiène ou de leurs mélanges, de préférence d'éthylène et de butylène.

7. Dispositif pour la libération de substances selon la revendication 5, **caractérisé en ce que** les polymères sont construits à base de copolymères à blocs binaires (A-B) et/ou ternaires (ABA) avec une proportion de copolymères à blocs binaires inférieure à 80% en poids.

8. Dispositif pour la libération de substances selon la revendication 5, **caractérisé en ce que** la proportion totale en styrène dans le système polymère est inférieure à 35% en poids, de préférence de 5% en poids à 30% en poids.

9. Dispositif pour la libération de substances selon au moins l'une quelconque des revendications précédentes, **caractérisé en ce que** le dispositif est adhésif, de préférence auto-adhésif.

10. Dispositif pour la libération de substances selon au moins une revendication précédente, **caractérisé en ce que** le dispositif est une masse adhésive en masse fondue à chaud présentant une proportion de copolymères à blocs de SEPS d'au moins 3% en poids, en particulier constituée par

a) 3% en poids à 90% en poids de copolymères à blocs,
b) 5% en poids à 80% en poids d'agents poisseux, tels que les résines hydrocarbonées aliphatiques, les résines terpéniques hydrogénées, les résines hydrocarbonées hydrogénées, les résines de cyclopentadiène, les résines de styrène-α-méthylstyrène et les polyisobutylènes de bas poids moléculaire,
c) moins de 60% en poids de plastifiant, tels que les huiles de paraffine, les huiles hydrocarbonées aliphatiques, les cires et les esters et les alcool-éthers d'acides gras,
d) moins de 15% en poids d'additifs,
e) moins de 5% en poids de stabilisateurs,
f) moins de 20% en poids d'adjuvants pour la libération de la ou des substances actives,
f) moins de 60% en poids de substance active ou de substances actives.

11. Dispositif pour la libération de substances selon au moins une revendication précédente, **caractérisé en ce que** la température de transition vitreuse dynamique de la phase d'élastomère de la matrice auto-adhésive, à une fréquence de 0,1 rad/s, est inférieure à 15°C, de préférence de -3°C à -30°C, de manière tout particulièrement préférée de -9°C à -25°C.

12. Dispositif pour la libération de substances selon au moins l'une quelconque des revendications précédentes, **caractérisé en ce que** le dispositif est appliqué partiellement sur un matériau support, en particulier par impression matricielle, thermosérigraphie, impression thermoflexographique ou par héliogravure.

13. Dispositif pour la libération de substances selon au moins l'une quelconque des revendications précédentes, **caractérisé en ce que** le dispositif est pulvérisé sur un matériau support.

14. Dispositif pour la libération de substances selon au moins l'une quelconque des revendications précédentes, **caractérisé en ce que** le dispositif est appliqué sous forme de calottes polygéométriques sur le matériau support.

15. Dispositif pour la libération de substances selon au moins l'une quelconque des revendications précédentes, **caractérisé en ce que** le dispositif est appliqué sur le matériau support à un poids surfacique supérieur à 15 g/m$^2$, de préférence entre 90 g/m$^2$ et 400 g/m$^2$, de manière tout particulièrement préférée entre 130 g/m$^2$ et 300 g/m$^2$.

16. Dispositif pour la libération de substances selon au moins l'une quelconque des revendications précédentes, **ca-**

**ractérisé en ce que** la proportion en % de la surface revêtue par le dispositif sur le matériau support est d'au moins 20%, de préférence de 40% à 60%, de manière tout particulièrement préférée de 70% à 95%.

17. Dispositif pour la libération de substances selon au moins l'une quelconque des revendications précédentes, **caractérisé en ce que** le matériau support revêtu par le dispositif présente une perméabilité à l'air supérieure à 1 $cm^3/(cm^2*s)$, de préférence supérieure à 15 $cm^3/(cm^2*s)$, de manière tout particulièrement préférée supérieure à 70 $cm^3/(cm^2*s)$, et une perméabilité à la vapeur d'eau supérieure à 500 $g/(m^2*24h)$, de préférence supérieure à 1000 $g/(m^2*24h)$, de manière tout particulièrement préférée supérieure à 2000 $g/(m^2*24h)$.

18. Dispositif pour la libération de substances selon au moins l'une quelconque des revendications précédentes, **caractérisé en ce que** le dispositif est stérilisé, de préférence par des rayons $\gamma$.

19. Dispositif pour la libération de substances selon au moins l'une quelconque des revendications précédentes, **caractérisé en ce que** le dispositif présente une adhérence d'au moins 1,5 N/cm, en particulier une adhérence entre 2,5 N/cm et 5 N/cm.